# EUROPÄISCHE PATENTSCHRIFT

(11) **EP 1 971 623 B1**
(45) Veröffentlichungstag und Bekanntmachung des Hinweises auf die Patenterteilung: **16.09.2009**
(21) Anmeldenummer: 06841179.2
(22) Anmeldetag: 27.12.2006
(51) Int. Cl.: C08G 18/02, C08G 18/79, C07C 267/00, C07D 229/00

(54) **VERFAHREN ZUR HERSTELLUNG FLÜSSIGER, LAGERSTABILER CARBODIIMID- UND/ODER URETONIMINGRUPPEN AUFWEISENDER ORGANISCHER ISOCYANATE**
METHOD FOR PRODUCING LIQUID, STORAGE-STABLE ORGANIC ISOCYANATES COMPRISING CARBODIIMIDE AND/OR URETONIMINE GROUPS
PROCEDE DE PRODUCTION D'ISOCYANATES ORGANIQUES PRESENTANT DES GROUPES DE CARBODIIMIDES ET/OU D'URETONIMINES, STABLES AU STOCKAGE ET LIQUIDES

(30) Priorität: 05.01.2006 DE 102006000822
(43) Veröffentlichungstag der Anmeldung: 24.09.2008
(73) Patentinhaber: Bayer MaterialScience AG, 51368 Leverkusen (DE)
(72) Erfinder: WERSHOFEN, Stefan, 41065 Mönchengladbach (DE); STEINWEGS, Marcus, 47829 Krefeld (DE)
(86) Internationale Anmeldenummer: PCT/EP2006/012553
(87) Internationale Veröffentlichungsnummer: WO 2007/077000

(56) Entgegenhaltungen:
- EP-A- 0 515 933
- US-A1- 2003 134 741
- US-B1- 6 362 247

## Beschreibung

Die Erfindung betrifft ein Verfahren zur Herstellung flüssiger, lagerstabiler Carbodiimid- (CD) und/oder Uretonimin- (UI) Gruppen aufweisender Isocyanatmischungen mit niedriger Farbzahl, die nach diesem Verfahren erhältlichen Isocyanatmischungen und deren Verwendung zur Herstellung von Abmischungen mit weiteren Isocyanaten bzw. zur Herstellung von Isocyanatgruppen enthaltenden Prepolymeren sowie von Polyurethankunststoffen, vorzugsweise Polyurethanschaumstoffen.

CD- und /oder UI-Gruppen aufweisende Isocyanatmischungen können in einfacher Weise mit den hoch wirksamen Katalysatoren aus der Phospholin-Reihe, insbesondere der Phospholinoxid-Reihe, nach den Verfahren gemäß US-A-2,853,473, US-A-6,120,699 und EP-A-515 933 hergestellt werden.

Die hohe katalytische Aktivität der Phospholinkatalysatoren, insbesondere der Phospholinoxidkatalysatoren, ist einerseits erwünscht, um die Carbodiimidisierungsreaktion unter schonenden Temperaturbedingungen anzustoßen, andererseits ist aber bis heute kein Verfahren bekannt, das eine wirksame Abstoppung der Phospholin-Katalyse bzw. der Phospholinoxid-Katalyse ohne Einschränkung gewährleistet. Die carbodiimidisierten Isocyanate neigen zur Nachreaktion, d.h. sie gasen infolge CO₂-Entwicklung aus. Dies führt dann besonders bei höheren Temperaturen zu einem Druckaufbau beispielsweise in den Lagerbehältern.

Es hat nicht an Versuchen gefehlt, eine wirksame Abstoppung der Phospholin-Katalyse zu finden. Derartige Stopper sind z.B. in den Patentschriften DE-A-25 37 685, EP-A-515 933, EP-A-609 698 und US-A-6,120,699 erwähnt und umfassen z.B. Säuren, Säurechloride, Chloroformiate, silylierte Säuren und Halogenide der Hauptgruppenelemente. Ein Abstoppen des Katalysators mit Säuren, die z.B. auch als Säurechloride vorliegen können, ist nicht ausreichend wirksam.

Nach der Lehre der EP-A-515 933 werden mittels Phospholin-Katalyse hergestellte CD/LTI-haltige Isocyanatmischungen mit mindestens der äquimolaren Menge, bevorzugt der 1-2fachen molaren Menge bezogen auf den eingesetzten Katalysator an z.B. Trimethylsilyltrifluormethansulfonat (TMST) abgestoppt. In der Praxis hat sich jedoch erwiesen, dass solchermaßen hergestellte CD/UI-enthaltende Isocyanate zur Herstellung von Prepolymeren, d.h. Umsetzungsprodukten von diesen CD/UI-enthaltenden Isocyanaten mit Polyolen, nur bedingt geeignet sind. Die entsprechend hergestellten Umsetzungsprodukte aus Polyolen und den CD/UI-modifizierten Isocyanaten neigen zum Ausgasen, was zu einem Druckaufbau in den Transportbehältem oder zum Schäumen bei der Handhabung derartiger Produkte führen kann.

Man kann dieses Problem dadurch umgehen, dass man die zum Abstoppen des Phospholin-Katalysators benutzte silylierte Säure analog der EP-A-515 933 in höheren molaren Äquivalenten (z.B. 5:1-10:1 bezogen auf den Katalysator) einsetzt. In der Praxis zeigt sich dann jedoch, dass die erhaltenen CD/UI modifizierten Isocyanate eine deutlich schlechtere Farbzahl aufweisen. Dies gilt dann auch für die hieraus hergestellten Prepolymere.

Dies gilt auch, wenn der Phospholinkatalysator mit Säuren vom Typ der Trifluormethansulfonsäure entsprechend der US-A-6,120,699 abgestoppt wird. Auch daraus hergestellte Prepolymere weisen eine erheblich erhöhte Farbzahl auf.

Bei der Herstellung von flüssigen, lagerstabilen Carbodiimid- (CD) und/oder Uretonimin- (UI) Gruppen aufweisenden Isocyanatmischungen werden z.T. deutliche Schwankungen der Reaktivität des eingesetzten Isocyanates und damit der erforderlichen Reaktionszeiten beobachtet. Einer unerwünschten Verlängerung der Reaktionszeit könnte z.B. durch Erhöhung der Reaktionstemperatur und/oder der Katalysatorkonzentration (und in Folge der Stoppermenge) begegnet werden. Damit wären aber verfahrens- und/oder sicherheitstechnische Risiken und/oder Qualitätsprobleme (z.B. erhöhte Farbwerte) verbunden.

Aufgabe der vorliegenden Erfindung war es daher, ein einfaches und wirtschaftliches Verfahren zur Herstellung flüssiger, lagerstabiler und heller Carbodiimid- und/oder Uretonimingruppen aufweisender Isocyanatmischungen zur Verfügung zu stellen, welches die angesprochenen Mängel nicht aufweist und zu flüssigen, lagerstabilen Isocyanatmischungen mit niedrigen Farbzahlen führt.

Die Erfindung betrifft ein Verfahren zur Herstellung Carbodiimid- und/oder Uretonimingruppen aufweisender organischer Isocyanate, bei dem ein oder mehrere organische Isocyanate mit einer Hazen-Farbzahl von ≤ 100 APHA, bevorzugt ≤ 50 APHA, mit Katalysatoren vom Phospholin-Typ teilweise carbodümidisiert werden, und anschließend die Carbodiimidisierungsreaktion abgestoppt wird, **dadurch gekennzeichnet, dass** die Carbodiimidisierung in Gegenwart eines silylierten Säureamids durchgeführt wird. Dadurch kann die erforderliche Reaktionszeit erniedrigt werden bzw. niedrig gehalten werden und/oder die erforderliche Katalysatormenge reduziert werden.

In dem erfindungsgemäßen Verfahren kann ein silyliertes Säureamid oder auch ein Gemisch mehrerer verschiedener silylierter Säureamide eingesetzt werden. Die Zugabe des silylierten Säureamids kann dabei zu dem Ausgangsisocyanat oder zum Reaktionsgemisch während der Carbodiimidisierung erfolgen. Das silylierte Säureamid wird dabei bevorzugt in Substanz, d.h. ohne Verdünnung, oder als Masterbatch, beispielsweise als Lösung des silylierten Säureamids im Ausgangsisocyanat oder bereits carbodiimidisiertem Isocyanat zugegeben.

Die Messung der Hazen-Farbzahl kann dabei gemäß DIN/EN/ISO 6271-2 (Entwurf September 2002) in Substanz gegen Wasser als Referenz bei einer Schichtdicke von 5 cm erfolgen. Als Messgerät kann z.B. ein Photometer Dr. Lange LICO 300 eingesetzt werden.

Selbstverständlich können auch organische Isocyanate mit einer höheren Farbzahl als Einsatzstoffe verwendet werden. In diesem Falle können allerdings die Vorteile hinsichtlich der günstigen Farbwerte nicht in vollem Umfang genutzt werden.

Die Erfindung betrifft auch die Carbodiimid- und/oder Uretonimingruppen aufweisenden organischen Isocyanate, die nach dem obengenannten Verfahren erhältlich sind. Diese Carbodiimid- und/oder Uretonimingruppen aufweisenden organischen Isocyanate sind bei Raumtemperatur und in Abhängigkeit vom CD- / UI-Gehalt und/oder vom eingesetzten Isocyanat bis hin zu tiefen Temperaturen (z.B. 0°C) flüssig.

Gegenstand der Erfindung ist auch die Verwendung der erfindungsgemäßen Carbodiimid- und/oder Uretonimingruppen aufweisenden organischen Isocyanate zur Herstellung von Abmischungen mit weiteren Isocyanaten bzw. zur Herstellung von Isocyanatgruppen enthaltenden Prepolymeren mit verbesserter Farbzahl.

Gegenstand der Erfindung ist schließlich auch die Verwendung der erfindungsgemäßen Carbodiimid- und/oder Uretonimingruppen aufweisenden organischen Isocyanate und der daraus hergestellten Isocyanat-Abmischungen und/oder Prepolymere mit verbesserter Farbzahl zur Herstellung von Polyurethankunststoffen.

Als Ausgangsmaterialien für das erfindungsgemäße Verfahren können beliebige organische Isocyanate mit einer Hazen-Farbzahl von ≤ 100 APHA, bevorzugt ≤ 50 APHA, eingesetzt werden. Vorzugsweise wird das erfindungsgemäße Verfahren jedoch zur Carbodiimidisierung von organischen Diisocyanaten verwendet, die in der Polyurethan-Chemie eingesetzt werden können.

Selbstverständlich können auch organische Isocyanate mit einer höheren Farbzahl als Einsatzstoffe verwendet werden. In diesem Falle können allerdings die Vorteile hinsichtlich der günstigen Farbwerte nicht in vollem Umfang genutzt werden.

Geeignete Isocyanate sind z.B. aromatische, araliphatische, aliphatische und/oder cycloaliphatische Diisocyante und/oder Polyisocyanate..

Als Vertreter der aliphatischen und/oder cycloaliphatischen Diisocyanate sind beispielhaft zu nennen Isophorondiisocyanat, Hexamethylendüsocyanat und Dicyclohexylmethandüsocyanat (jeweils die reinen Isomeren sowie beliebige Isomerengemische).

Als Vertreter der araliphatischen Diisocyante sind beispielhaft zu nennen die verschiedenen Isomeren der Xylidendiisocyante.

Geeignet sind insbesondere aromatische Di- und polyisocyanate wie Toluylendiisocyanat und Di- und Polyisocyanate der Diphenylmethanreihe.

Geeignet sind insbesondere:
- Aromatische Diisocyanate wie 2,4- und/oder 2,6-Diisocyanatotoluol (TDI), 2,2'-, 2,4'-und/oder 4,4'-Diisocyanatodiphenylmethan (MDI) bzw. beliebige Gemische derartiger aromatischer Diisocyanate,
- Di- und Polyisocyanatgemische der Diphenylmethanreihe mit einem Gehalt an monomeren Diisocyanatodiphenylmethan-Isomeren von 80 bis 100 Gew.-% und einem Gehalt an höher als difunktionellen Polyisocyanaten der Diphenylmethanreihe von 0 bis 20 Gew.-%, wobei sich die Diisocyanatodiphenylmethan-Isomeren zu 0 bis 100 Gew.-% aus 4,4'-Diisocyanatodiphenylmethan, 100 bis 0 Gew.-% aus 2,4'-Diisocyanatodiphenylmethan und zu 0 bis 8 Gew.-% aus 2,2'-Diisocyanatodiphenylmethan zusammensetzen, wobei sich die genannten Prozentsätze zu 100 Gew.-% ergänzen.

Als Ausgangsmaterialien bevorzugte organische Isocyanate sind insbesondere aromatische Diisocyanate wie 2,4- und/oder 2,6-Diisocyanatotoluol (TDI), 2,2'-, 2,4'- und/oder 4,4'-Diisocyanatodiphenylmethan (MDI) bzw. beliebige Gemische derartiger aromatischer Diisocyanate. Besonders bevorzugt sind 2,2'-, 2,4'- und/oder 4,4'-Diisocyanatodiphenylmethan (MDI) bzw. beliebige Gemische derartiger aromatischer Diisocyanate, wobei die Summe an 2,2'-, 2,4'-und/oder 4,4'-Diisocyanatodiphenylmethan im Ausgangsmaterial (organisches Isocyanat) mindestens 85 Gew.-% beträgt, und wobei sich die Diisocyanatodiphenylmethan-Isomeren zu 0 bis 100 Gew.-% aus 4,4'-Diisocyanatodiphenylmethan, 100 bis 0 Gew.-% aus 2,4'-Diisocyanatodiphenylmethan und zu 0 bis 8 Gew.-% aus 2,2'-Diisocyanatodiphenylmethan zusammensetzen, wobei sich die genannten Prozentsätze zu 100 Gew.-% ergänzen. Ganz besonders bevorzugt sind 2,2'-, 2,4'- und/oder 4,4'-Diisocyanatodiphenylmethan (MDI) bzw. beliebige Gemische aromatischer Diisocyanate, wobei die Summe an 2,2'-, 2,4'- und/oder 4,4'-Diisocyanatodiphenylmethan im Ausgangsmaterial (organisches Isocyanat) mindestens 90 Gew.-% beträgt, und wobei sich die Diisocyanatodiphenylmethan-Isomeren zu 0 bis 100 Gew.-% aus 4,4'-Diisocyanatodiphenylmethan, 100 bis 0 Gew.-% aus 2,4'-Diisocyanatodiphenylmethan und zu 0 bis 8 Gew.-% aus 2,2'-Diisocyanatodiphenylmethan zusammensetzen, wobei sich die genannten Prozentsätze zu 100 Gew.-% ergänzen. Insbesondere ganz besonders bevorzugt sind 2,2'-, 2,4'- und/oder 4,4'-Diisocyanatodiphenylmethan (MDI) bzw. beliebige Gemische aromatischer Diisocyanate, wobei die Summe an 2,2'-, 2,4'- und/oder 4,4'-Diisocyanatodiphenylmethan im Ausgangsmaterial (organisches Isocyanat) mindestens 99 Gew.-% beträgt, und wobei sich die Diisocyanatodiphenylmethan-Isomeren zu 0 bis 100 Gew.-% aus 4,4'-Diisocyanatodiphenylmethan, 100 bis 0 Gew.-% aus 2,4'-Diisocyanatodiphenylmethan und zu 0 bis 8 Gew.-% aus 2,2'-Diisocyanatodiphenylmethan zusammensetzen, wobei sich die genannten Prozentsätze zu 100 Gew.-% ergänzen.

Das erfindungsgemäße Verfahren wird in Gegenwart von Katalysatoren vom Phospholin-Typ durchgeführt. Die Katalysatoren vom Phospholin-Typ sind beispielsweise aus EP-A-515 933 und US-A-6,120,699 bekannt. Typische Beispiele dieser Katalysatoren sind beispielsweise die aus dem Stand der Technik bekannten Gemische der Phospholinoxide der Formel:

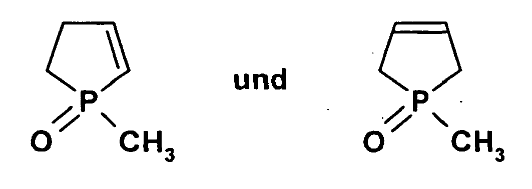

Die Menge des eingesetzten Katalysators hängt von der Qualität und/oder der Reaktivität der Ausgangsisocyanate ab. Die jeweils notwendige Katalysatormenge lässt sich daher am einfachsten in einem Vorversuch bestimmen.

Durch den Einsatz von silylierten Säureamiden wird erreicht, dass die Reaktivität des Ausgangsisocyanats erhöht wird. Dies kann z.B. ursächlich dadurch erfolgen, dass sie der reaktivitätsvermindernden Wirkung von potentiell HCl abspaltenden Nebenkomponenten im Ausgangsisocyanat entgegenwirken. Aber auch andere Wirkmechanismen sind möglich.

Geeignete silylierte Säureamide sind z.B. silylierte Carbonsäureamide oder silylierte Carbaminate, wie z.B. N-Trimethylsilylacetamid, N,O-Bis(trimethylsilyl)acetamid, oder Trimethylsilyl-N-trimethylsilylcarbaminat oder Gemische daraus. Die genannten Verbindungen werden nur als Beispiele angesehen; geeignete silylierte Säureamide sind nicht auf die genannten Verbindungen beschränkt.

Das silylierte Säureamid oder das Gemisch mehrerer verschiedener silylierter Säureamide kann unmittelbar vor, gleichzeitig mit oder auch erst nach der Zugabe des Katalysators zugegeben werden. Vorzugsweise wird das silylierte Säureamid erst nach der Zugabe des Katalysators, d.h. während der Carbodimidisierung, zugegeben. Der beste Zeitpunkt der Zugabe kann in einem einfachen Vorversuch ermittelt werden und liegt bevorzugt vor Erreichen von 50%, besonders bevorzugt vor Erreichen von 30% und ganz besonders bevorzugt vor Erreichen von 20% des insgesamt gewünschten Umsatzes an Isocyanat.

Die optimale Einsatzmenge des silylierten Säureamids kann ebenfalls in einem einfachen Vorversuch ermittelt werden und beträgt bevorzugt ≤ 1000 ppm, besonders bevorzugt ≤ 250 ppm und ganz besonders bevorzugt ≤ 100 ppm, bezogen auf das Gewicht des eingesetzten Isocyanat.

Die Zugabe des silylierten Säureamids kann also zu dem Ausgangsisocyanat oder zum Reaktionsgemisch während der Carbodiimidisierung erfolgen. Das silylierte Säureamid wird dabei bevorzugt in Substanz, d.h. ohne Verdünnung, oder als Masterbatch, beispielsweise als Lösung des silylierten Säureamids im Ausgangsisocyanat oder bereits carbodiimidisierten Isocyanat zugegeben.

Der Einsatz des silylierten Säureamids resultiert in einer höheren Reaktivität bezüglich der Carbodiimidisierungsreaktion, wodurch entweder die erforderliche Reaktionszeit und/oder die erforderliche Katalysatormenge reduziert werden kann.

Die Carbodiimidisierungsreaktion wird üblicherweise im Temperaturbereich zwischen 50 bis 150°C, vorzugsweise von 60 bis 100°C, durchgeführt. Jedoch sind auch deutlich höhere Reaktionstemperaturen möglich (bis zu ca. 280°C). Die optimale Reaktionstemperatur richtet sich nach der Art der Ausgangsisocyanate und / oder des eingesetzten Katalysators und kann in einem einfachen Vorversuch ermittelt werden.

Die Carbodiimidisierungsreaktion wird im allgemeinen bei Erreichen eines Carbodiimidisierungsgrades (Carbodiimidisierungsgrad ist der Prozentsatz der carbodiimidisierten Isocyanatgruppen bezogen auf die Gesamtmenge der in Ausgangsisocyanat vorliegenden Isocyanatgruppen) von 3 bis 50 %, vorzugsweise 5 bis 30 %, abgebrochen.

Der Carbodiimidisierungsgrad kann während der Durchführung des erfindungsgemäßen Verfahrens durch Bestimmung des NCO-Wertes z.B., mittels dem Fachmann an sich bekannter Titration oder mittels online-Verfahren bestimmt werden. Ein geeignetes online-Verfahren ist z.B. die Nahinfrarot- oder die Mittelinfrarot-Analytik.

Der Carbodiimidisierungsgrad kann während der Durchführung des erfindungsgemäßen Verfahrens ebenfalls z.B. an der Menge des im Reaktorgemisch entweichenden Kohlendioxids erkannt werden. Diese volumetrisch bestimmbare Kohlendioxidmenge gibt somit zu jedem Zeitpunkt Auskunft über den erreichten Carbodiimidisierungsgrad.

Darüber hinaus können grundsätzlich auch andere geeignete, dem Fachmann bekannte offline- oder online-Methoden der Prozessverfolgung eingesetzt werden.

Zum Beenden der Carbodiimidisierungsreaktion wird bevorzugt mindestens die äquimolaren Menge, besonders bevorzugt der 1 - 20 fache molare Überschuss, ganz besonders bevorzugt der 1 - 10 fache molare Überschuss, bezogen auf den Katalysator, eines Abstoppers, bevorzugt Trimethylsilyltrifluormethansulfonat (TMST) oder eines Alkylierungsmittels oder eines Gemisches der genannten Abstopper eingesetzt. Bevorzugt wird dabei ein Alkylierungsmittel oder Trimethylsilyltrifluormethansulfonat (TMST) als einziger Abstopper eingesetzt.

Bevorzugte Alkylierungsmittel sind Ester der Trifluormethansulfonsäure, Ester anorganischer Säuren (bevorzugt starker anorganischer Säuren) oder Trialkyloxoniumverbindungen.

Das Reaktionsprodukt der Carbodiimidisierung kann Farbstabilisatoren enthalten, wie sie üblicherweise Isocyanaten zugesetzt werden. Dabei ist der Zeitpunkt des Zusatzes nicht kritisch. Die Farbstabilisatoren können entweder dem als Ausgangmaterial verwendeten Isocyanat vor der Carbodiimidisierung zugesetzt werden, oder dem Reaktionsprodukt nach vollendeter Umsetzung. Es ist ebenfalls möglich, Farbstabilisatoren sowohl dem Ausgangsmaterial als auch dem Reaktionsprodukt zuzugeben. Derartige Stabilisatoren sind generell dem Fachmann bekannt und umfassen z.B. Substanzen aus der Gruppe der sterisch gehinderten Phenole, der Phosphorigsäureester oder der sterisch gehinderten Amine. Die Farbstabilisatoren können jeweils für sich allein oder im Gemisch mit anderen Vertretern der gleichen oder verschiedener Substanzgruppen eingesetzt werden. Die Mengen der eingesetzten Farbstabilisatoren bewegen sich in der dem Fachmann bekannten Größenordnung, üblicherweise im Bereich von 100 ppm bis 10000 ppm für die Einzelsubstanz bzw. das Gemisch, bezogen auf das als Ausgangsmaterial verwendete Isocyanat bzw. das Reaktionsprodukt der Carbodiimidisierung.

Isocyanatgruppen enthaltende Prepolymere werden durch Umsetzung der nach dem erfindungsgemäßen Verfahren hergestellten Carbodiimid- und/oder Uretonimingruppen aufweisenden organischen Isocyanate mit in der Polyurethanchemie üblichen Polyolen erhalten. Geeignete Polyole sind sowohl einfache mehrwertige Alkohole des Molekulargewichtsbereiches 62 bis 599 g/mol, vorzugsweise 62 bis 300 g/mol, wie z.B. Ethylenglykol, Trimethylolpropan, Propandiol-1,2, Butandiol-1,2 oder Butandiol-2,3, Hexandiol, Octandiol, Dodecandiol und/oder Octadecandiol, insbesondere jedoch höhermolekulare Polyetherpolyole und/oder Polyesterpolyole der aus der Polyurethanchemie an sich bekannten Art mit Molekulargewichten von 600 bis 8000 g/mol, vorzugsweise 800 bis 4000 g/mol, die mindestens zwei, in der Regel 2 bis 8, vorzugsweise 2 bis 4 primäre und/oder sekundäre Hydroxylgruppen aufweisen. Beispiele derartiger Polyole sind in der US-PS 4 218543, Kolonne 7, Zeile 29 bis Kolonne 9, Zeile 32 beschrieben.

Die Vorteile des erfindungsgemäßen Verfahrens sind augenscheinlich: Durch die Anwesenheit eines silylierten Säureamids während der Carbodiimidisierung wird die Reaktivität des Reaktionsgemisches erhöht und/oder vereinheitlicht. Dadurch kann die erforderliche Reaktionszeit erniedrigt werden bzw. niedrig gehalten werden und/oder die erforderliche Katalysatormenge reduziert werden. Sowohl die Carbodiimid- und/oder Uretonimingruppen-haltigen Isocyanate als auch die daraus hergestellten Prepolymere weisen zudem eine gute Lagerstabilität und eine helle Farbe auf.

Diese Carbodiimid- und/oder Uretonimingruppen aufweisenden organischen Isocyanate und die daraus durch Umsetzung mit Polyolen hergestellten Prepolymere stellen wertvolle Ausgangsmaterialien zur Herstellung von Polyurethankunststoffen durch Umsetzung mit Polyolen (z.B. mit Polyetherpolyolen oder Polyesterpolyolen) nach dem Isocyanat-Polyadditionsverfahren dar.

### Beispiele

### Ausgangsprodukte:

- Desmodur 44M^{®}, Bayer AG (4,4'- Diphenylmethandiisocyanat, NCO-Gehalt: 33,6 Gew.-%)
- Katalysator vom Phospholinoxid-Typ: technisches Gemisch aus 1-Methyl-1-oxo-1-phosphacyclopent-2-en und 1-Methyl-1-oxo-1-phosphacyclopent-3-en, 1 Gew.-%ig in Toluol
- Stopper: Trifluormethansulfonsäureethylester (TFMSEE) bzw. Trimethylsilyltrifluormethansulfonat (TMST)

Allgemeine Vorschrift zur Herstellung des Carbodiimid- und/oder Uretonimingruppen aufweisenden organischen Isocyanates:

10 kg technisches 4,4'-MDI (Desmodur 44M^{®},der Bayer MaterialScience AG) mit einer Hazen Farbzahl von < 15 APHA, das 750 ppm 3,5-Di-*tert*-butyl-4-hydroxytoluol enthält, werden unter N₂/Rühren auf ca. 90°C erhitzt. Anschließend wird die in der Tabelle angegebene Menge Katalysatorlösung zugegeben, um die gewünschte Katalysatormenge zu erreichen. Das Reaktionsgemisch wird mit der entsprechenden Menge an silyliertem Säureamid versetzt (Zeitpunkt, Substanz und Menge siehe Tabelle; A: N,O-Bis(trimethylsilyl)acetamid; B: Trimethylsilyl-N-trimethylsilylcarbaminat). Das Reaktionsgemisch wird unter N₂/Rühren bis zum Erreichen des erwünschten NCO-Gehaltes auf ca. 95°C erhitzt. Danach wird die Carbodiimidisierung durch Zugabe des jeweiligen Stoppers (Trifluormethansulfonsäureethylester (TFMSEE) bzw. Trimethylsilyltrifluormethansulfonat (TMST); siehe Tabelle) abgestoppt und 1 Stunde nachgerührt.

Die Ergebnisse sind in der nachstehenden Tabelle zusammengefasst.

Die Messung der Hazen-Farbzahl erfolgt gemäß DIN/EN/ISO 6271-2 (Entwurf September 2002) in Substanz gegen Wasser als Referenz bei einer Schichtdicke von 5 cm. Als Messgerät kann z. B. ein Photometer Dr. Lange LICO 300 eingesetzt werden.

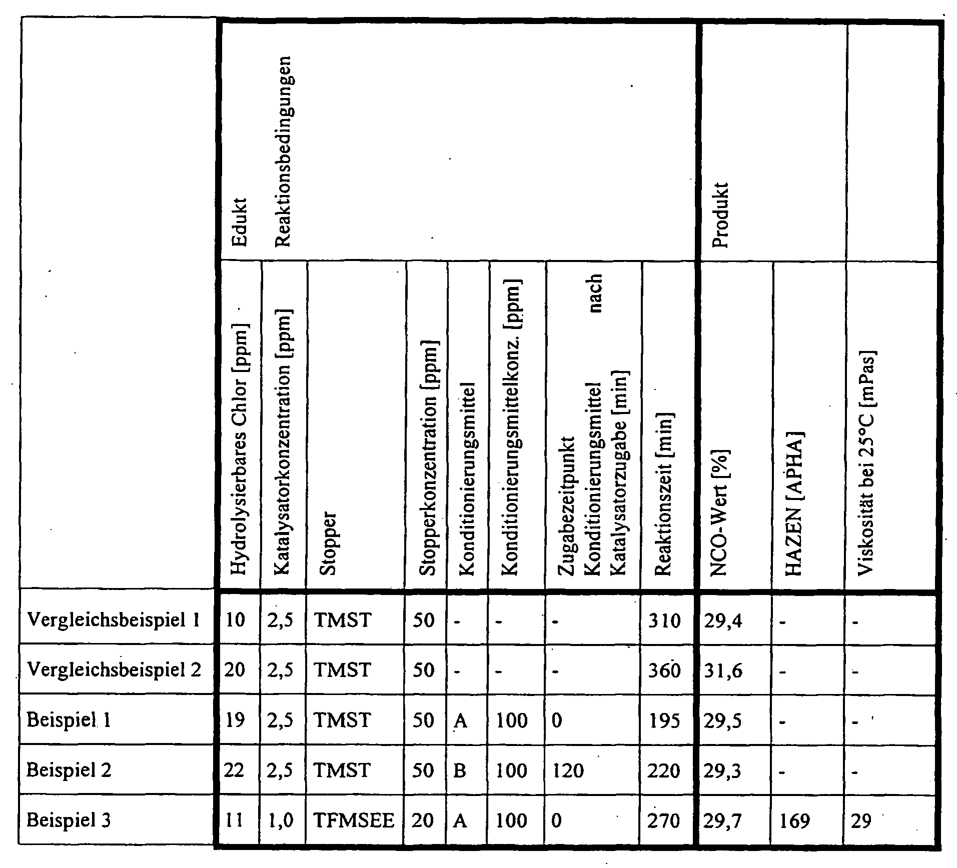

Vergleichsbeispiele 1 und 2 verdeutlichen den Einfluss des erhöhten Gehalts an hydrolysierbarem Chlor auf die Reaktivität bzw. die Reaktionszeit.

In den erfindungsgemäßen Beispielen 1 und 2 wird eine gegenüber Vergleichsbeispiel 2 verbesserte Reaktivität errecht, die bei gleicher Konzentration an eingesetztem Katalysator zu kürzeren Reaktionszeiten in den erfindungsgemäßen Beispielen 1 und 2 führt.

Trotz einer geringerer Konzentration an eingesetztem Katalysator im erfindungsgemäßen Beispiel 3 wird eine gegenüber Vergleichsbeispiel 1 sogar verkürzte Reaktionszeit benötigt. Dies demonstriert die durch die Zugabe des silylierten Säureamids bewirkte Erhöhung der Reaktivität des als Ausgangsisocyanat eingesetzten 4,4'- Diphenylmethandüsocyanat.

Dabei erreichen die erhaltenen Produkte ein gutes Farb-Niveau (HARZEN).

## Patentansprüche

1. Verfahren zur Herstellung Carbodiimid- und/oder Uretonimingruppen aufweisender organischer Isocyanate, bei dem ein oder mehrere organische Isocyanate mit einer Hazen-Farbzahl von ≤ 100 APHA, bevorzugt ≤ 50 APHA, mit Katalysatoren vom Phospholin-Typ teilweise carbodümidisiert werden, und anschließend die Carbodiimidisierungsreaktion abgestoppt wird, **dadurch gekennzeichnet, dass** die Carbodiimidisierung in Gegenwart eines silylierten Säureamids durchgeführt wird.

2. Verfahren nach Anspruch 1, **dadurch gekennzeichnet, dass** als silyliertes Säureamid ein silyliertes Carbonsäureamid eingesetzt wird.

3. Verfahren nach Anspruch 1, **dadurch gekennzeichnet, dass** als silyliertes Säureamid ein silyliertes Carbaminat eingesetzt wird.

4. Verfahren nach Anspruch 1, **dadurch gekennzeichnet, dass** als silyliertes Säureamid N-Trimethylsilylacetamid, N,O-Bis(trimethylsilyl)acetamid, oder Trimethylsilyl-N-trimethylsilylcarbaminat oder Gemische daraus eingesetzt werden.

5. Verfahren nach einem der Ansprüche 1 bis 4, **dadurch gekennzeichnet, dass** das silylierte Säureamid unmittelbar vor, gleichzeitig mit oder nach der Zugabe des Katalysators zugegeben wird.

6. Verfahren nach einem der Ansprüche 1 bis 5, **dadurch gekennzeichnet, dass** das silylierte Säureamid in Konzentrationen von ≤ 1000 ppm, bevorzugt ≤ 250 ppm, besonders bevorzugt ≤ 100 ppm, bezogen auf das Gewicht des eingesetzten Isocyanats, vorliegt.

7. Verfahren nach Anspruch 6, **dadurch gekennzeichnet, dass** das silylierte Säureamid in Substanz zugegeben wird.

8. Verfahren nach Anspruch 6, **dadurch gekennzeichnet, dass** das silylierte Säureamid als Masterbatch in Ausgangsisocyanat oder bereits carbodiimidisiertem Isocyanat zugegeben wird.

## Claims

1. Process for the preparation of organic isocyanates containing carbodiimide and/or uretonimine groups, in which one or more organic isocyanates having a Hazen colour number of ≤ 100 APHA, preferably ≤ 50 APHA, are partly carbodiimidized with catalysts of the phospholine type and the carbodiimidization reaction is subsequently terminated, **characterized in that** the carbodiimidization is carried out in the presence of a silylated acid amide.

2. Process according to claim 1, **characterized in that** a silylated carboxylic acid amide is employed as the silylated acid amide.

3. Process according to claim 1, **characterized in that** a silylated carbamate is employed as the silylated acid amide.

4. Process according to claim 1, **characterized in that** N-trimethylsilylacetamide, N,O-bis(trimethylsilyl)acetamide, or trimethylsilyl N-trimethylsilylcarbamate or mixtures thereof are employed as the silylated acid amide.

5. Process according to one of claims 1 to 4, **characterized in that** the silylated acid amide is added immediately before, at the same time as or after the addition of the catalyst.

6. Process according to one of claims 1 to 5, **characterized in that** the silylated acid amide is present in concentrations of ≤ 1,000 ppm, preferably ≤ 250 ppm, particularly preferably ≤ 100 ppm, based on the weight of the isocyanate employed.

7. Process according to claim 6, **characterized in that** the silylated acid amide is added in substance.

8. Process according to claim 6, **characterized in that** the silylated acid amide is added as a masterbatch in the starting isocyanate or already carbodiimidized isocyanate.

## Revendications

1. Procédé de préparation d'isocyanates organiques présentant des radicaux carbodiimide et/ou urétonimine, dans lequel un ou plusieurs isocyanates organiques avec un indice de trouble de ≤ 100 APHA, de préférence ≤ 50 APHA, sont soumis à une carbodiimidation partielle avec des catalyseurs de type phospholine, et ensuite la réaction de carbodiimidation est arrêtée, **caractérisé en ce que** la carbodiimidation est réalisée en présence d'un amide d'acide silylé.

2. Procédé selon la revendication 1, **caractérisé en ce que** l'on met en oeuvre comme amide d'acide silylé, un amide d'acide carboxylique silylé.

3. Procédé selon la revendication 1, **caractérisé en ce que** l'on met en oeuvre comme amide d'acide silylé, un carbaminate silylé.

4. Procédé selon la revendication 1, **caractérisé en ce que** l'on met en oeuvre comme amide d'acide silylé, le N-triméthylsilylacétamide, le N,Obis(triméthylsilyl)acétamide, ou le triméthylsilyl-N-triméthylsilylcarbaminate, ou des mélanges de ceux-ci.

5. Procédé selon l'une des revendications 1 à 4, **caractérisé en ce que** l'on ajoute l'amide d'acide silylé, directement avant, simultanément à ou après l'addition du catalyseur.

6. Procédé selon l'une des revendications 1 à 5, **caractérisé en ce que** l'amide d'acide silylé est présent en des concentrations ≤ 1000 ppm, de préférence ≤ 250 ppm, de manière particulièrement préférée ≤ 100 ppm, sur base du poids de l'isocyanate mis en oeuvre.

7. Procédé selon la revendication 6, **caractérisé en ce que** l'amide d'acide silylé est ajouté tel quel.

8. Procédé selon la revendication 6, **caractérisé en ce que** l'amide d'acide silylé est ajouté sous forme d'un mélange maître, dans l'isocyanate de départ ou dans l'isocyanate déjà carbodiimidé.
